(19) Europäisches Patentamt
European Patent Office
Office européen des brevets

(11) **EP 1 850 752 B1**

(12) **FASCICULE DE BREVET EUROPEEN**

(45) Date de publication et mention
de la délivrance du brevet:
**06.04.2011 Bulletin 2011/14**

(21) Numéro de dépôt: **06724834.4**

(22) Date de dépôt: **03.02.2006**

(51) Int Cl.:
*A61B 5/20* (2006.01)   *A61M 25/00* (2006.01)

(86) Numéro de dépôt international:
**PCT/EP2006/050651**

(87) Numéro de publication internationale:
**WO 2006/084824 (17.08.2006 Gazette 2006/33)**

(54) **SONDE POUR EXAMEN URODYNAMIQUE**

URODYNAMISCHE UNTERSUCHUNGSSONDE

URODYNAMIC EXAMINATION PROBE

(84) Etats contractants désignés:
**DE ES GB IT**

(30) Priorité: **08.02.2005 FR 0501246**

(43) Date de publication de la demande:
**07.11.2007 Bulletin 2007/45**

(73) Titulaire: **Peters Surgical**
**93000 Bobigny (FR)**

(72) Inventeur: **GUICHARD, Jean**
**27290 Ecaquelon (FR)**

(74) Mandataire: **Texier, Christian et al**
**Cabinet Régimbeau**
**20, rue de Chazelles**
**75847 Paris Cedex 17 (FR)**

(56) Documents cités:
**EP-A- 0 878 166    WO-A-2004/089216**
**GB-A- 2 123 300    US-A- 4 073 287**

**Description**

L'invention concerne une sonde pour examen urologique du type urodynamique muni d'un dispositif pour pratiquer cet examen.

L'examen urodynamique vise à étudier le comportement dynamique de la vessie et/ou de l'urètre.

A cet effet, les systèmes à prise de pression à fluide disposent d'une sonde qui mesure les pressions vésicale et urétrale.

Le principe de mesure de la pression vésicale consiste à remplir la vessie avec de l'eau et à mesurer la pression intravésicale.

La prise de pression urétrale consiste à perfuser un liquide à débit constant au travers un orifice latéral d'une sonde introduite dans l'urètre.

On mesure ensuite les variations de pression en amont en fonction de la position de la sonde le long de l'urètre. Ce type de mesure a fait notamment l'objet d'une étude complète par Braun et Wickham apporté dans l'article intitulé « The uretral pressure profile » et paru dans la revue the Bristish Journal of Urology (41-211.216, en 1969).

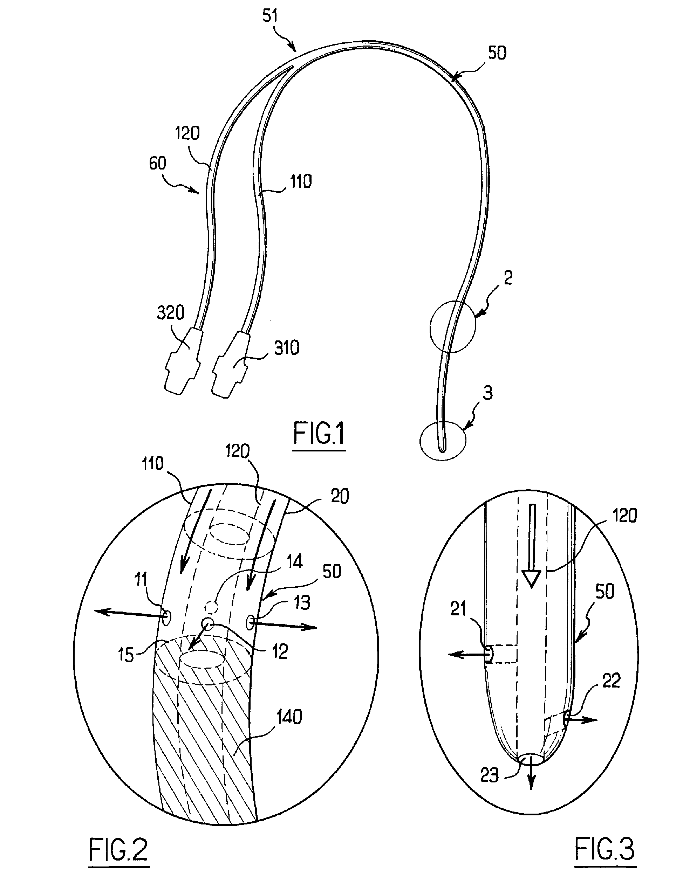

En référence à la figure 1, est représentée une vue générale d'une sonde selon l'état de la technique. Cette sonde comporte une partie distale 50 destinée à être introduite dans l'urètre du patient, et une partie proximale 60 destinée à être raccordée aux appareils de mesure, de perfusion, ou autres dispositifs utilisés par le médecin, par l'intermédiaire d'embases 310, 320. La sonde comprend ici un tube urétral 110 et un tube vésical 120, le tube vésical 120 étant destiné à prendre la pression vésicale du patient, le tube urétral 110 étant destiné à véhiculer le fluide perfusé pour réaliser une prise de pression urétrale ou à véhiculer le fluide pour remplir la vessie.

La partie distale 50 comprend les parties distales des deux tubes urétral 110 et vésical 120, le tube vésical 120 s'étendant à l'intérieur du tube urétral 110, l'insertion du tube vésical 120 dans le tube urétral 110 ayant été réalisée en amont (au niveau de la zone 51). La partie distale 50 est agencée de sorte à pouvoir être introduite dans l'urètre du patient et à ce que sa zone d'extrémité 3 puisse parvenir à l'intérieur de la vessie.

En référence à la figure 2, qui représente une vue en agrandissement de la zone 2 de la figure 1, l'espace situé entre le tube vésical 120 et le tube urétral 110 (dans lequel doit circuler le fluide perfusé pour prise de pression urétrale) est fermé au niveau de la surface 15 au moyen d'un tube obturateur 140. La paroi du tube urétral 110 est en outre pourvu d'oeils 11, 12, 13 et 14 au voisinage de cette extrémité 15. Ainsi, lorsqu'un fluide est perfusé dans le tube urétral 120, celui-ci est évacué par les oeils 11, 12, 13 et 14.

En référence à la figure 3, qui est une vue en agrandissement de la zone 3 de la figure 1, est représentée la zone d'extrémité de la sonde. Les parois de la sonde sont pourvues au niveau de sa zone d'extrémité 3, d'un ou de plusieurs oeils 21, 22, 23 prévus pour réaliser la prise de pression vésicale avec un fluide présent dans le tube vésical 120.

Il est montré sur la figure 2 que cette sonde permet une prise de pression circonférentielle au tube urétral 110, répartissant de façon homogène la pression sur la circonférence de l'urètre, minimisant alors les pertes des charges pour un meilleur examen de l'urètre.

Le fonctionnement de cette sonde est le suivant :

Une première étape consiste à remplir la vessie par l'intermédiaire du tube urétral 110 en faisant « descendre » la sonde de sorte que la zone de prise de pression (représentée sur la figure 2) se trouve dans la vessie, puis à « remonter » la sonde de sorte que la zone de prise de pression se situe cette fois dans une zone de l'urètre déterminée et alors prendre la pression urétrale dans cette zone. La prise de pression vésicale étant alors assurée par l'intermédiaire du tube vésical 120.

Cette manipulation en va-et-vient de la sonde dans l'urètre du patient, provoque un frottement supplémentaire de la sonde contre les parois de l'urètre qui augmente le risque de traumatisme.

D'autre part, les mesures urodynamiques sont longues et nécessitent une manipulation supplémentaire consistant à déconnecter le tube urétral 110 du système permettant le remplissage vésical puis à le reconnecter sur des appareils de mesure de pression urétrale pour réaliser alors la prise de pression urétrale.

Enfin, cette sonde n'autorise pas une prise de pression urétrale et un remplissage vésical simultanés, puisque le tube urétral 110 a pour fonction non seulement de permettre une prise de pression urétrale, mais aussi un remplissage vésical.

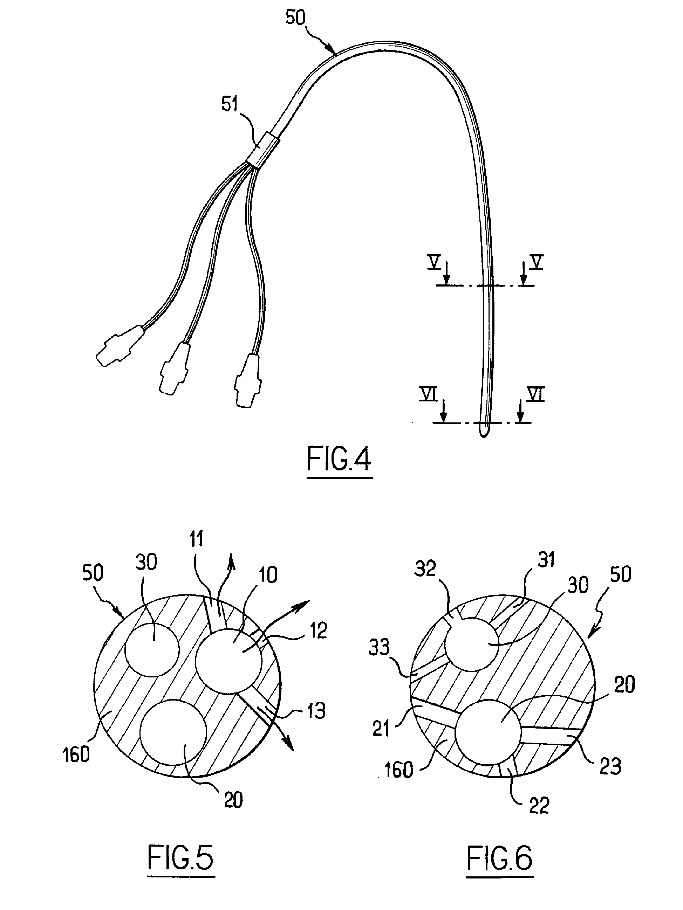

En référence aux figures 4, 5 et 6, est représentée une autre sonde de l'état de la technique (voir en particulier le document US 5 385 563) tentant d'améliorer la situation par rapport à celle précédemment discutée.

Pour atteindre cet objectif, la partie distale 50 est constituée d'un unique tube 160 plein pourvu d'un canal urétral 10 et de deux canaux vésicaux 20 et 30, les deux canaux vésicaux 20 et 30 s'étendant jusqu'à la zone d'extrémité de la partie distale 50 de la sonde, telle que représentée sur la figure 6 qui est une vue en coupe selon VI-VI de la figure 4. Les deux canaux vésicaux 20 et 30 sont respectivement munis d'oeils 21, 22, 23 et 31, 32, 33 dans la zone d'extrémité de la sonde afin de réaliser respectivement le remplissage vésical et la prise de pression vésicale pour la mesure urodynamique. Le canal urétral 10 a une longueur adaptée pour qu'une perfusion du fluide par son intermédiaire soit possible sur une zone de l'urètre une fois la sonde en place.

Des tubes prolongateurs 210, 220, 230 sont alors fixés sur les sorties respectives des canaux 110, 120, 130, dans la partie proximale 60 de la sonde, pour être alors connectés à des appareils de mesure et de pression (par l'intermédiaire des embases 310, 320, 330)

Cette sonde permet de réaliser des mesures de pressions vésicale et urétrale simultanées, de diminuer le temps de mesure, et de réduire les risques traumatiques liés au frottement de la sonde contre les parois de l'urètre.

Cependant, en référence à la figure 5 qui est une vue en coupe selon V-V de la figure 4, la prise de fluide urétrale n'est ici réalisée que sur une portion de la circonférence de l'urètre du fait du voisinage avec les canaux vésicaux 20 et 30 qui empêchent de prévoir des oeils à partir du canal urétral 10 vers l'extérieur sur une portion importante de la circonférence de la sonde.

Ainsi, cette autre sonde de l'état de la technique mesure des pressions urétrales seulement sur une portion circonférentielle limitée de l'urètre, et ne permet donc pas de rendre compte de la réactivité des surfaces urétrales le long de la circonférence d'une section urétrale donnée. Cette asymétrie ou latéralisation dans les mesures peut ainsi fausser les résultats de celles-ci. Il serait toutefois possible de mesurer la pression sur une section d'urètre en tournant la sonde autour de l'axe de l'urètre d'un nombre de fois suffisant pour réaliser 360°, ce sont cependant des mesures déconseillées pour le risque traumatique important de l'urètre, que cela pourrait engendrer chez le patient.

En outre, cette configuration augmente les risques de perturbation des mesures par perte de charge du fait d'un diamètre hydraulique faible dans ce type de canal.

Le document EP 0878166 décrit une sonde urodynamique qui requiert la mise en place d'un dispositif d'interconnexion de sources de fluide et de capteurs sur la sonde à l'aide d'un commutateur de débit et de robinets. Néanmoins, ces éléments complémentaires induisent des défauts de mesure des pressions urétrale et vésicale.

La sonde décrite dans le document GB 2123300 présente plusieurs canaux disjoints. Ces canaux permettent une connexion de la sonde aux capteurs et à une source de fluide simultanément. Un seul de ces canaux est dédié à une mesure de la pression urétrale. La présence de ces multiples canaux rend impossible l'ouverture de l'un d'eux sur la circonférence de la sonde. Ainsi la prise de mesure de la pression urétrale ne peut être qu'effectuée sur une portion circonférentielle limitée de l'urètre.

Un objectif de la présente invention est de répartir de façon homogène la prise de pression urétrale sur une circonférence de l'urètre.

Un autre objectif de l'invention est de minimiser les problèmes de perte de charge.

Un autre objectif est de pouvoir réaliser simultanément un remplissage vésical et une mesure de pression vésicale et urétrale.

Un autre objectif est de minimiser les frottements de la sonde contre les parois de l'urètre.

Un autre objectif est de minimiser le temps de mesure urodynamique.

Un autre objectif est de prévoir une sonde suffisamment rigide pour faciliter l'introduction de la sonde dans l'urètre, minimiser les risques de blocage au niveau du sphincter vésical, et diminuer l'amortissement des pressions.

A cet effet, l'invention propose, selon un premier aspect, une sonde destinée à un examen urodynamique d'un patient, comprenant une partie proximale, et une partie distale s'étendant longitudinalement de sorte à être logée dans l'urètre du patient et sur une longueur suffisante pour qu'une zone d'extrémité soit localisée dans la vessie du patient, la partie distale comprenant trois canaux internes indépendants, caractérisée en ce qu'elle comprend, dans sa partie distale, un tube intérieur et un tube extérieur, au moins une partie du tube intérieur s'étendant longitudinalement dans le tube extérieur de sorte à définir entre eux un premier canal, les deux autres canaux s'étendant dans le tube intérieur.

D'autres caractéristiques de cette sonde sont présentées ci-après :

- la surface intérieure du tube extérieure et la surface externe du tube intérieur sont en moyenne suffisamment éloignées l'une de l'autre pour qu'un fluide perfusé puisse circuler dans le premier canal avec une pression apte à servir une mesure urodynamique ;
- le tube extérieur est agencé de sorte que le premier canal débouche, en opération, au niveau d'une zone de l'urètre de sorte qu'un fluide perfusé dans le premier canal puisse exercer une pression sur les parois de l'urètre apte à servir une mesure urodynamique, et le tube intérieur est agencé de sorte que les deux autres canaux débouchent au niveau de la zone d'extrémité de la partie distale ;
- le premier canal débouche par au moins un oeil traversant une paroi du tube extérieur ;
- le premier canal débouche par plusieurs oeils traversant la paroi du tube extérieur, et ces oeils sont localisés de sorte à répartir une prise de pression sur la totalité de la circonférence de ladite zone de l'urètre ;
- le tube intérieur est agencé de sorte que le deuxième canal puisse véhiculer un fluide pour remplir la vessie, que le troisième canal puisse déboucher dans la vessie de sorte qu'un fluide perfusé dans le troisième canal puisse exercer une pression sur un fluide remplissant la vessie apte à servir une mesure urodynamique ;
- le troisième canal débouche par au moins un oeil traversant une paroi du tube intérieur ;
- le tube intérieur comporte une paroi interne s'étendant sur tout le tube intérieur de sorte à définir lesdits deux autres canaux ;

- la sonde comprend en outre un tube intermédiaire s'étendant au moins en partie entre le tube intérieur et le tube extérieur et dont une extrémité ferme le premier canal ;
- le tube intermédiaire s'étend jusqu'à l'extrémité du tube intérieur ;
- le tube extérieur se prolonge sur une partie du tube intermédiaire, et la sonde comprend en outre un tube terminal s'étendant sur l'autre partie du tube intermédiaire en prolongement du tube extérieur ;
- le tube extérieur se prolonge sur l'ensemble du tube intermédiaire ;
- le tube intermédiaire s'étend sur une partie du tube intérieur, le tube extérieur s'étend jusqu'à l'extrémité du tube intermédiaire, et la sonde comprend un tube terminal fixé sur la partie libre du tube intérieur ;
- la partie proximale comprend un tube urétral, un premier tube vésical et un deuxième tube vésical, étant respectivement en communication avec ledit premier canal, un deuxième canal et un troisième canal ;
- la sonde comprend en outre des moyens de connexion reliés en bout de la partie proximale de la sonde, ces moyens de connexion étant agencés de sorte à connecter la sonde avec des appareils de perfusion, de mesure urodynamique ou d'autres dispositifs ;
- la sonde est en PVC, silicone, polyuréthane, élastomère, PET, ou PEBA (Polyéther Bloc Amide) ;
- sa partie distale a un diamètre moyen compris entre charrière 7 et charrière 12 ;
- la sonde est plongée dans une enceinte d'eau et est dimensionnée de sorte à présenter une erreur relative inférieure à 5 % pour des débits de perfusion dans le premier canal entre 0 et 2 ml/min, et des pressions statiques entre 0 et 200 cm d'eau ;
- la sonde est configurée de sorte à avoir un temps de réaction aux pressions urétrales inférieur ou de l'ordre de 1 seconde.

Selon un deuxième aspect, l'invention propose un prolongateur de ladite sonde comprenant deux canaux de prolongation aptes à venir se connecter à l'extrémité de la partie proximale de la sonde, agencés pour prolonger deux desdits trois canaux de la sonde destinés respectivement à des mesures urétrales et vésicales, et ayant été calibrés de sorte que l'ensemble qu'ils forment avec ces deux canaux ait les propriétés décrites dans les deux paragraphes précédents.

Une autre caractéristique de ce prolongateur est présentée ci-après :

- il comprend un troisième tube, indépendant des deux canaux de prolongation précités, apte à venir se connecter à l'extrémité de la partie proximale du troisième canal.

Selon un troisième aspect, l'invention propose un ensemble de sonde constitué de ladite sonde et dudit prolongateur.

D'autres aspects, buts et avantages de cette invention sortiront de la description qui suit, qui est purement illustrative et non limitative, et qui sera lue en regard des dessins annexés sur lesquels :

Les figures 1, 2 et 3 représentent une première sonde de l'état de la technique, la figure 1 étant une vue générale et les figures 2 et 3 étant des agrandissements respectifs des zones 2 et 3 de la figure 1.
Les figures 4, 5 et 6 représentent une deuxième sonde de l'état de la technique, la figure 4 représentant une vue générale de la sonde, et les figures 5 et 6 des vues en coupe respectives selon les sections transversales V-V et VI-VI.
Les figures 7, 8a, 8b et 9a, 9b représentent des vue d'une sonde selon l'invention, la figure 7 étant une vue générale de la sonde, les figures 8a et 9a étant des agrandissements respectifs des zones 8 et 9 de la sonde selon la figure 7, les figures 8b et 9b étant des vues en coupe respectives selon les sections transversales VIII-VIII et IX-IX.
Les figures 10a à 10f représentent différentes étapes de réalisation d'une sonde selon l'invention.
Les figures 11, 12 et 13 représentent trois sondes selon l'invention dans leur partie distale, selon une vue transversale à la paroi interne du tube intérieur.
La figure 14 représente une étape finale de réalisation d'une sonde selon l'invention.
Les figures 15, 16 et 17 représentent des mesures de pression en sortie du canal urétral et du canal vésical de sondes selon l'invention.

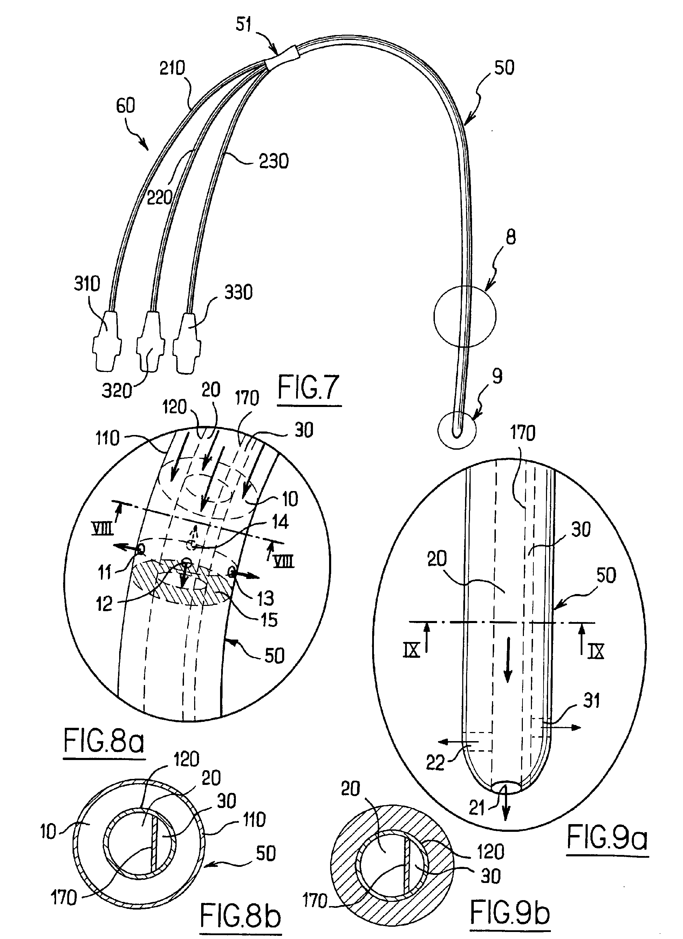

Ci-après est décrit, en référence aux figures 7, 8a, 8b et 9a, 9b, une sonde pour examen urodynamique préférée selon l'invention.

En référence à la figure 7, la sonde comprend deux parties principales :

- une partie distale 50 ayant l'apparence extérieure d'un tube unique s'étendant longitudinalement de sorte à être logée dans l'urètre du patient et sur une longueur suffisante pour qu'une zone d'extrémité 9 puisse être localisée dans la vessie du patient. La partie distale 50 comprend deux tubes 110 et 120, ainsi que trois canaux internes indépendants, dont un canal urétral 10, un premier canal vésical 20 et un troisième canal vésical 30 (comme montré par exemple sur les figures 8a et 8b) ;

- une partie proximale 60, raccordée aux trois canaux de la partie distale 50 par l'intermédiaire d'un moyen de raccord 51, qui constitue au moins une partie de la liaison entre les appareils utilisés par le praticien et la partie distale 50. Lesdits appareils utilisés peuvent comprendre des moyens permettant de détecter et de mesurer des pressions fluidiques perfusées dans les différents canaux de la sonde, ainsi que des moyens pour perfuser les différents canaux de la sonde.

La partie proximale 60 peut être constituée de trois tubes d'extension 210, 220 et 230 destinés à venir allonger respectivement les canaux 10, 20 et 30 de la partie distale de la sonde. Des moyens de connexion ou embases 310, 320 et 330 peuvent en outre être prévus à l'extrémité libre de la partie proximale 60 afin de faciliter la connexion des trois tubes d'extension 210, 220 et 230 avec lesdits appareils utilisés.

Le mode de raccordement (au niveau de la zone 51 de la figure 7) des trois tubes d'extension 210, 220 et 230 à l'embout des trois canaux 10, 20 et 30 peut être réalisé par tout moyen connu de l'homme du métier.

En référence à la figure 8a, est représentée la zone 8 de la sonde au niveau de laquelle la prise de pression urétrale doit être réalisée lorsque la partie distale 50 est mise en place dans l'urètre. Cet agrandissement de la zone 8 de la figure 7 permet d'observer les différents éléments compris dans cette partie de la sonde, à savoir :

- un tube extérieur 110 dont la surface extérieure constitue la surface externe de la sonde dans sa partie distale 50 et dont la surface intérieure représente la surface extérieure du canal urétral 10 ;
- un tube intérieur 120 situé, dans cette portion de la partie distale 50 de la sonde, à l'intérieur du tube extérieur 110 de sorte à laisser entre sa surface externe et la surface interne du tube extérieur 110 un espace représentant le canal urétral 10. Bien entendu, on agence les dimensions des deux tubes pour que cet espace soit suffisant pour qu'un fluide perfusé puisse y circuler avec une pression apte à servir une mesure urétrale lors d'un examen urodynamique.

La paroi du tube extérieur 110 est pourvue d'un ou plusieurs oeils 11, 12, 13 et 14 localisés dans la zone 8 de sorte à faire communiquer le canal urétral 10 avec le milieu extérieur à la sonde (i.e. l'urètre une fois la sonde mise en place), de sorte que ce fluide perfusé exerce une pression apte à servir une mesure urétrale lors d'un examen urodynamique. Préférentiellement, on choisit un nombre et un mode de répartition des oeils 11, 12, 13, 14 de sorte à répartir de façon homogène la pression du fluide sur la circonférence des parois urétrales voisines. On peut de cette façon minimiser les pertes de charge dans la sonde, et améliorer la qualité des mesures.

Dans un mode de réalisation particulier (non représenté), on pourra prévoir de répartir aussi les oeils sur la longueur du tube extérieur 110, de sorte à réaliser aussi des prises de pression urétrales à différents niveaux dans l'urètre.

En outre, la sonde comprend un moyen permettant d'obturer (au niveau de la surface d'obturation 15) le canal urétral 10 au voisinage des oeils 11, 12, 13, 14 de sorte que les uniques voies de sortie pour le fluide perfusé dans le canal urétral 10 soient les oeils 11, 12, 13, 14.

Le tube intérieur 120 est pourvu d'une paroi interne 170 s'étendant sur toute la longueur du tube intérieur 120 et séparant de façon étanche l'intérieur du tube intérieur 120 en deux parties 20 et 30 définissant deux canaux vésicaux 20 et 30.

Cette paroi interne 170 permet notamment de définir deux canaux 20, 30, pour une section minimale du tube intérieur 120. En minimisant la section du tube intérieur 120, est aussi minimisée la section du tube extérieur 110, et permet ainsi une structure de sonde selon l'invention qui puisse s'insérer dans l'urètre du patient par les voies naturelles, sans intervention chirurgicale et en minimisant la douleur chez le patient.

En référence aux figures 9a et 9b, ce tube intérieur 20 s'étend en éloignement de la partie proximale 60 de sorte à être suffisamment long pour, lorsque la sonde est mise en position dans l'urètre, pouvoir déboucher dans la vessie.

Le premier canal vésical 20 débouche ainsi dans la vessie par l'intermédiaire d'un ou plusieurs oeils 21, 22 traversant l'extrémité distale de la sonde en prolongement du premier canal vésical 20 (i.e. oeil 21 sur la figure 9a) et/ou traversant transversalement la paroi longitudinale de la zone d'extrémité 9 de la sonde (i.e. oeil 22).

Le deuxième canal vésical 30 débouche préférentiellement par un ou plusieurs oeils 31 traversant une paroi longitudinale de la zone d'extrémité 9 de la sonde.

La zone d'extrémité 9 de la sonde est agencée de sorte que l'unique voie de sortie vers l'extérieur pour le fluide perfusé dans le deuxième canal vésical 30 est l'oeil 31.

La partie distale 50 de la sonde peut par exemple avoir un diamètre extérieur moyen compris entre charrière 7 et charrière 12.

Ainsi, cette sonde permet une mesure simultanée d'une pression urétrale via un fluide perfusé dans le canal urétral 10, et d'une pression vésicale via un fluide perfusé dans le deuxième canal vésical 30.

En outre, il n'est pas nécessaire de modifier la position de la sonde dans l'urètre une fois que celle-ci est mise en place, à l'exception du mouvement nécessaire pour le retrait de la sonde, évitant ainsi tout risque supplémentaire de traumatisme sur les parois de l'urètre.

Enfin, la configuration particulière de la sonde selon l'invention comprenant trois canaux et deux tubes permet de minimiser la section de la partie distale 50 de la sonde par rapport par exemple au cas où on aurait défini les trois canaux 10, 20, 30 par trois tubes différents.

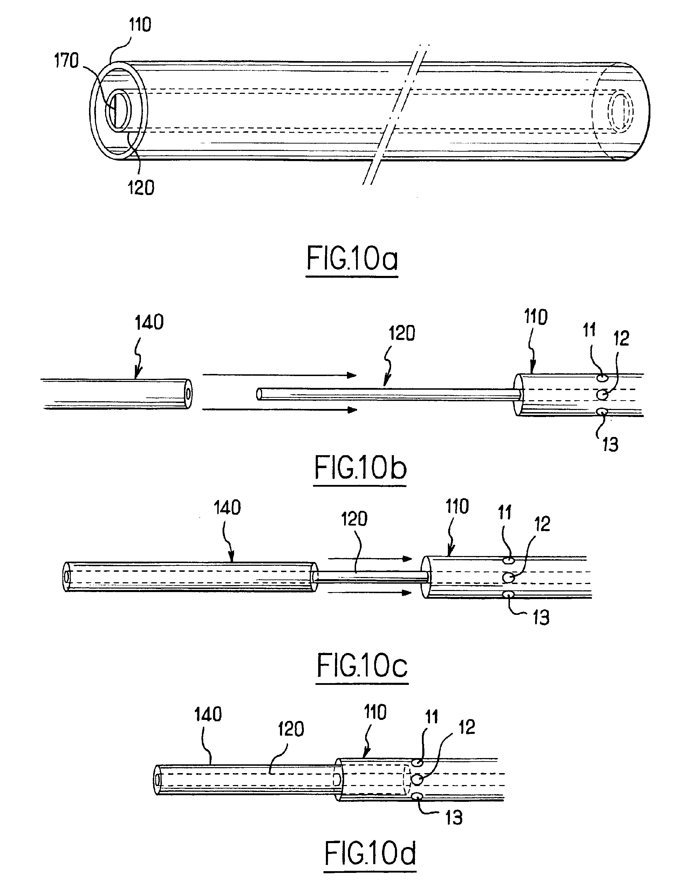

En référence aux figures 10a à 10f, est présenté un exemple de mode de réalisation d'une sonde selon l'invention.

En référence à la figure 10a, on se procure un tube extérieur 110 et un tube intérieur 120 de sorte que la section du tube extérieur 110 soit choisie pour que le tube extérieur puisse être introduit dans l'urètre d'un patient pour un examen urodynamique.

Le diamètre intérieur du tube extérieur 110 et le diamètre extérieur du tube intérieur 120 sont choisis de sorte que, une fois le tube intérieur 120 enfilé dans le tube extérieur 110, un espace défini entre ces deux surfaces soit suffisant pour permettre à un fluide qui y serait perfusé de circuler à une pression adaptée pour un examen urodynamique.

Le tube intérieur 120 comprend une paroi interne 170 s'étendant sur toute la longueur et divisant alors (comme on l'a vu précédemment) deux canaux intérieurs vésicaux 20 et 30. Ce type de tube, existant sur le marché, peut par exemple être obtenu par extrudage.

Le diamètre intérieur de ce tube intérieur 120 et la disposition de la paroi 170 à l'intérieur de ce tube intérieur 120 sont choisis de sorte que le deuxième canal vésical 30 puisse véhiculer un fluide et permettre de transmettre une pression statique vésicale suffisante lors d'un examen urodynamique, et de sorte que le premier canal vésical 20 ait une section suffisante pour remplir la vessie suffisamment rapidement sans à-coup.

De préférence avant l'enfilage du tube intérieur 120 dans le tube extérieur 110, les oeils 11, 12, 13, 14 sont pratiqués dans les parois longitudinales du tube extérieur 110 localisés dans une zone du tube au niveau de laquelle on souhaite réaliser les mesures urétrales. Ces oeils 11, 12, 13, 14 peuvent être par exemple pratiqués par poinçonnage, par exemple montés à rotatif, ou au moyen d'un laser adapté. On répartira de façon adaptée les oeils 11, 12, 13, 14 sur la circonférence du tube extérieur 110 de sorte que, une fois la sonde mise en place dans l'urètre, la prise de pression urétrale soit répartie de façon homogène. Ainsi, par exemple, si les oeils 11, 12, 13, 14 sont au nombre de quatre, on pourra séparer deux oeils adjacents de 90° sur la circonférence du tube extérieur 110.

Selon le mode de réalisation de la sonde illustrée en référence aux figures 10a à 10f, on prévoit une longueur du tube extérieur 110 plus petite que celle du tube intérieur 120 de sorte à laisser une petite partie du tube extérieur 110 au-delà des oeils 11, 12, 13, 14, et une partie restante du tube intérieur 120 sortante (voir figure 10b).

En référence aux figures 10b, 10c et 10d, on prévoit un tube intermédiaire 140 de diamètre intérieur sensiblement égal au diamètre extérieur du tube intérieur 120, de diamètre extérieur sensiblement égal au diamètre intérieur du tube extérieur 110, et d'une longueur sensiblement voisine de la distance séparant les oeils 11, 12, 13, 14 de l'extrémité distale du tube intérieur 120. Ce tube est alors enfilé sur la partie libre du tube intérieur 120 pour venir ensuite encombrer l'espace libre laissé entre le tube extérieur 110 et le tube intérieur 120 jusqu'à ce que l'extrémité du tube intermédiaire 140 arrive au voisinage des oeils 11, 12, 13, 14. La partie restante de l'espace laissé entre le tube extérieur 110 et le tube intérieur 120, ainsi obturée à une extrémité par le tube intermédiaire 140, définit alors le canal urétral 10. Le tube intermédiaire 140 est enfin fixé aux tubes extérieur 110 et/ou intérieur 120 par des moyens connus de l'homme du métier, tels que par exemple du cyclohexanone.

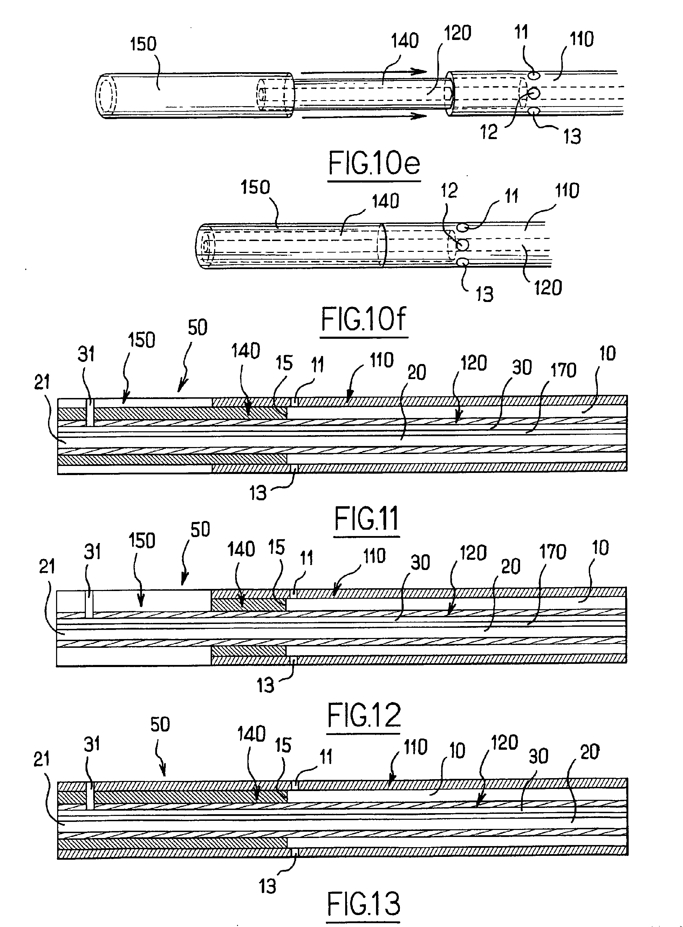

En référence aux figures 10e et 10f, on choisit un tube terminal 150 dont le diamètre intérieur est sensiblement identique au diamètre extérieur du tube intermédiaire 140, dont le diamètre extérieur est sensiblement égal au diamètre extérieur du tube extérieur 110, et dont la longueur est sensiblement identique à la longueur de la partie du tube intermédiaire 140 sortant du tube extérieur 110. Une fois que le tube terminal 150 est enfilé sur la partie restante du tube intermédiaire 140 jusqu'à venir en butée sur l'extrémité du tube extérieur 120, il est alors fixé sur le tube intermédiaire 140 par des moyens de fixation conventionnels qui peuvent être identiques à ceux utilisés lors de la fixation du tube intermédiaire 140 sur le tube intérieur 120 et/ou extérieur 110.

La partie distale 50 de la sonde alors obtenue est représentée en référence à la figure 11, après qu'un oeil 31 ait été pratiqué dans la paroi de la sonde afin de pouvoir faire communiquer le deuxième canal vésical 30 avec l'extérieur dans la zone d'extrémité de la sonde.

En référence à la figure 12, une autre sonde selon l'invention y est présentée, dans laquelle, le tube intermédiaire 140 ne s'étend ici pas au-delà du tube extérieur 110 (contrairement à ce qui était représenté sur la figure 10d), et dans lequel le tube terminal 150 a un diamètre intérieur sensiblement identique au diamètre extérieur du tube intérieur 120 est alors fixé sur le tube intérieur 110 (contrairement au mode de réalisation précédemment évoqué en référence aux figures 10e et 10f).

En référence à la figure 13, est représenté une autre sonde selon l'invention, dans laquelle on prévoit un tube extérieur 110 de la même longueur que le tube intérieur 120 et dans lequel n'est prévu aucun tube terminal 150. En effet, dans cette troisième sonde selon l'invention, aucun renfort au moyen d'un tube terminal 150 n'est pas nécessaire étant donné que cette fonction est ici remplie par le tube extérieur 110 et le tube intermédiaire 140. D'autre part, cette troisième sonde selon l'invention, ne présente aucune jonction entre un tube terminal 150 et le tube extérieur 110, contrairement aux deux premières sondes selon l'invention, ce qui permet d'avoir une surface extérieure de la sonde complètement uniforme et lisse.

Ces trois sondes selon l'invention présentent dans leur partie terminale des renforts adaptés (i.e. tube intermédiaire 140 et/ou tube terminal 150) qui vont permettre à la sonde de faciliter son introduction dans l'urètre, de limiter les déformations lors de son guidage dans l'urètre et dans la vessie, de pouvoir passer les barrières naturelles telles que par exemple le sphincter urétral, et de diminuer l'amortissement des pressions par la sonde.

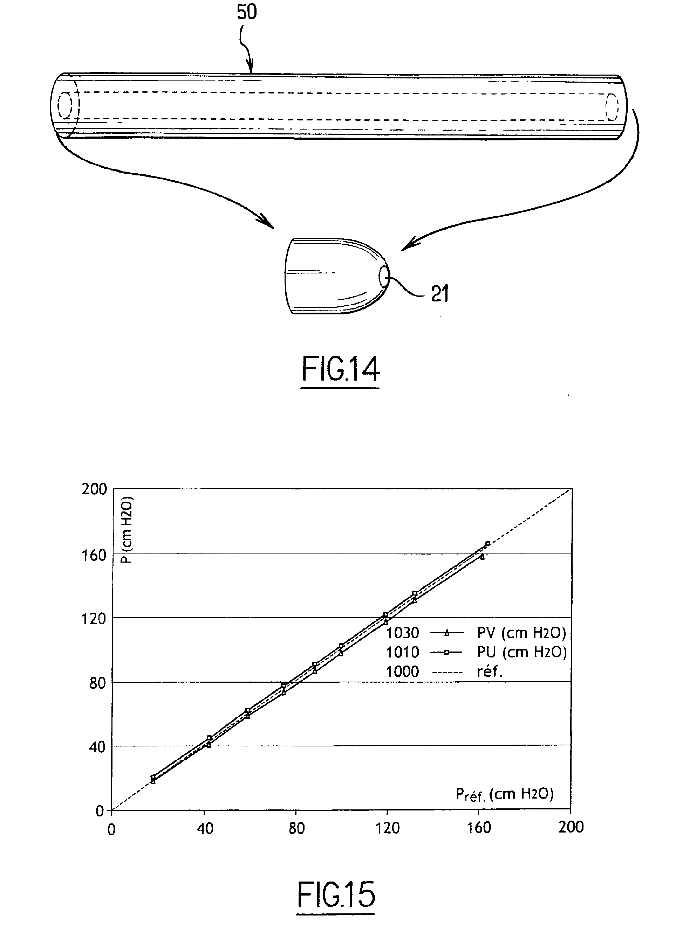

Enfin, en référence à la figure 14, on pourra avantageusement prévoir d'émousser au moins une extrémité de la partie distale 50 de la sonde de sorte à faciliter l'introduction et/ou le retrait de cette partie distale 50 de l'urètre.

La sonde selon l'invention est configurée, dimensionnée de sorte à améliorer le confort pour le patient lors de l'examen urodynamique, et en outre à augmenter la justesse, la qualité de la mesure, ainsi que la rapidité et la réactivité de la sonde à transmettre la mesure.

En particulier, il est important de dimensionner la sonde de sorte à éviter des surpressions créées par un écoulement dans les canaux urétral 10 et vésical 30, et donc à diminuer la perte de charge dans ces canaux lors des mesures, pour les valeurs de débit de perfusion couramment employées (de l'ordre de 0 à 5 ml/min typiquement pour le canal urétral 10 et de l'ordre de 0 ml/min pour le deuxième canal vésical 30).

La perte de charge dépend principalement du débit de l'écoulement, du diamètre intérieur du canal considéré et de la longueur du canal considéré comprenant à la fois la partie du canal située dans la partie distale 50 de la sonde et dans la partie proximale 60 de la sonde.

En outre, il convient de limiter les amortissements des pressions dans le canal qui peuvent être principalement dues à un manque de rigidité de la sonde et au matériau choisi pour les tubes.

A cet effet, on pourra choisir des duretés significatives pour les différents tubes constituant la sonde, telle qu'une dureté de tubes extérieur 110 et intérieur 130 en PVC comprise entre 80 et 95 shores, et une dureté comprise entre 70 et 95 shores pour le tube intermédiaire 140.

Afin de calibrer des sondes, la Demanderesse a alors réalisé des mesures de pression et de temps de réponse pour les deux sondes suivantes décrites dans le tableau 1.

Les sondes 1 et 2 sont en outre réalisées principalement en PVC, silicone, polyuréthane, élastomère, ou PET (Polyéthylène téréphtalate) ou PEBA (polyéther Bloc Amide).

Le côté proximal 60 de la sonde comprend tel que représenté sur la figure 1, des tubes d'extension urétral 210, et vésicaux 220 et 230 connectés chacun aux sorties respectives des canaux 10, 20 et 30 et se terminant chacun par des moyens de connexion de type embases 310, 320, 330 respectivement permettant de brancher chaque canal (urétral, premier vésical et deuxième vésical) à une source de liquide et à un capteur de pression. Ces derniers sont reliés électriquement à un appareil enregistreur non représenté. Des tests de calibration en milieu neutre ont alors été réalisés par la Demanderesse pour trouver les configurations et dimensionnements de la sonde appliquant notamment le moins de pertes de charge et un minimum d'amortissements.

Les tests ont consisté à plonger l'extrémité distale de la sonde dans une enceinte contenant de l'eau, représentant alors la vessie du patient après remplissage par l'intermédiaire du premier canal vésical 20, à remplir d'eau le premier canal vésical 20, ainsi qu'à faire communiquer le deuxième canal vésical 30 ainsi que le canal urétral 10, eux aussi remplis d'eau, avec l'enceinte.

On perfuse alors le canal urétral 10 et éventuellement le deuxième canal vésical 30 à des débits déterminés.

On exerce alors une pression déterminée sur l'eau contenue dans le récipient et on enregistre la pression à l'extrémité proximale des tubes d'extension urétral 210 et vésical 230, au moyen desdits capteurs disposés à leur niveau.

Ces tests préliminaires, dans un milieu non in vivo, permettent alors de calibrer la sonde et déterminer les dimensions, les caractéristiques et les configurations particulières de la sonde pour atteindre un rapport d'amplitude des pressions en sortie du canal urétral 10 et en sortie du deuxième canal vésical 30 le plus proche possible de 1.

Tableau 1

| Sondes | Diamètre de la sonde (en Charrières) | Position des oeils à partir de l'extrémité distale (en mm) | | Section des différents canaux | | | Longueur des différents canaux | | |
|---|---|---|---|---|---|---|---|---|---|
| | | Oeils urétraux 11, 12, 13, 14 | Oeil vésical 31 | Section du canal urétral 10 (en mm$^2$) | Section du 2$^{ème}$ canal vésical 30 (en mm$^2$) | Section du 1$^{er}$ canal vésical 20 (en mm$^2$) | Longueur du canal urétral 10 (en mm) | Longueur du 2$^{ème}$ canal vésical 30 (en mm) | Longueur du 1$^{er}$ canal vésical 20 (en mm) |
| Sonde n°1 | 12 | 71±1 | 7 ± 1 | 3,70 | 1,1 | 2,2 | 326 (+ 70 mm de tube d'extension 210) | 388 (+ 70 mm de tube d'extension 230) | 395 (+140mm de tube d'extension 220) |
| Sonde n° 2 | 9 | 71± 1 | 7 ± 1 | 1,95 | 0,65 | 1,25 | 326 | 388 | 395 |
| * Section du canal urétral | | | | | | | | | |

$$10 = \frac{\pi}{4} * [(\text{Diamètre intérieur du tube extérieur } 110)^2 - (\text{Diamètre extérieur du tube intérieur } 120)^2]$$

Il est reconnu que si l'erreur dans ce rapport est inférieure à 5 % en valeur absolue, la sonde sera considérée comme ayant une perte de charge et un amortissement suffisamment faibles pour être considérée comme satisfaisante.
En effet, cette erreur est principalement due à des pertes de charges et à l'amortissement relatifs à la sonde elle-même et aux matériaux dont ses tubes sont constitués.
Ainsi, la Demanderesse a réalisé des tests sur les sondes nos 1 et 2 susmentionnées pour des pressions exercées sur l'eau contenue dans l'enceinte comprises dans la gamme allant de 0 à 200 cm d'eau, pour des taux de perfusion dans le canal urétral 10 de 1 ml/min et 2 ml/min, et pour un taux de perfusion nul dans le deuxième canal vésical 30.
L'ensemble des résultats est présenté dans le tableau ci-après :

| Sonde | Perfusion urétrale | Voie vésicale à une pression de 200 cm d'eau | Voie urétrale à une pression de 200 cm d'eau |
|---|---|---|---|
| Sonde n° 1 | 1 ml/min | 0,49% | 0,49% |
| | 2 ml/min | | 0,50 % |
| Sonde n° 2 | 1 ml/min | 0,84% | 0,84% |
| | 2 ml/min | | 2,12 % |

Des courbes représentant les pressions mesurées en fonction des pressions exercées, après une période transitoire de stabilisation des valeurs, sont présentées sur :

- la figure 15, lorsque la sonde n° 2 est perfusée dans le canal urétral 10 à environ 1 ml/min ;

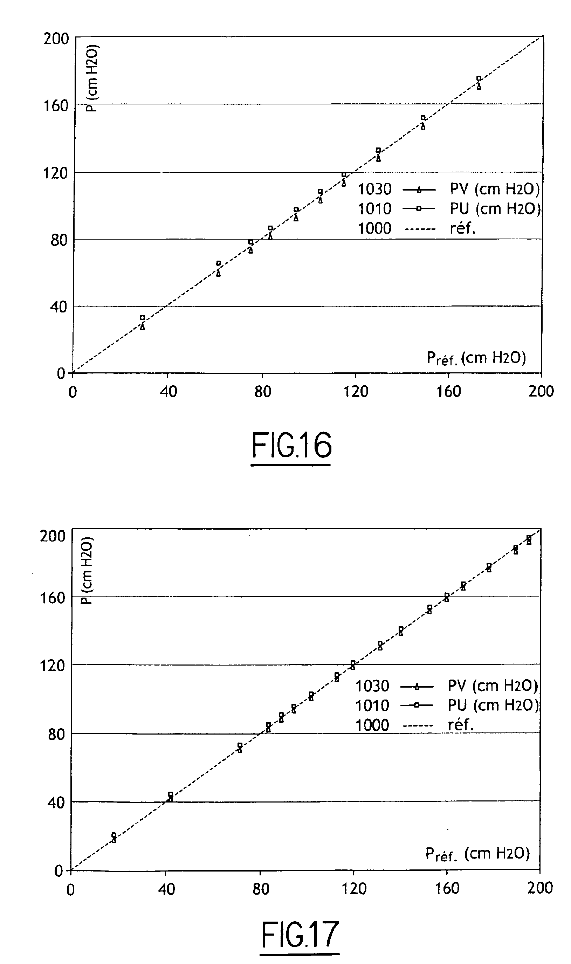

- la figure 16, lorsque la sonde n° 2 est perfusée dans le canal 10 à un taux de perfusion d'environ 2 ml/min ;
- la figure 17, lorsque la sonde n° 1 est perfusée dans le canal urétral 10 à un taux de perfusion d'environ 2 ml/min.

A 1 ml/min, les résultats obtenus sur la sonde n° 1 sont du même ordre.
Les courbes 1000, 1020 et 1030 représentent respectivement les pressions exercées sur l'eau du récipient, les pressions urétrales et les pressions vésicales en fonction de la pression de référence (i.e. la pression exercée sur l'eau du récipient).
En outre, la Demanderesse a réalisé des mesures de temps de réponse du canal urétral 10, lorsqu'est appliquée une pression déterminée sur l'eau contenue dans l'enceinte. Le principe consiste donc à appliquer cette pression à l'eau contenue dans l'enceinte pendant un temps déterminé et à mesurer le temps que met le canal urétral 10 pour transmettre 95 % de cette pression à sa sortie. Là encore, les mesures ont été effectuées pour la sonde 1 et la sonde 2
Il s'avère que les sondes n° 1 et 2 selon l'invention, permettent des temps de réponse à 95 % urétraux inférieurs ou de l'ordre de 1 s.
En outre, les erreurs relatives sont très inférieures à 5 % pour le canal urétral 10 et pour le deuxième canal vésical 30. Elles sont pratiquement toutes inférieures ou de l'ordre de 1 %.
Ces mesures ont donc mis en évidence que la sonde amortit peu la pression exercée sur l'enceinte d'eau (i.e. sur l'eau remplissant la vessie) et a une perte de charge limitée.
Ainsi, les longueurs et diamètres des canaux selon l'invention ont été avantageusement sélectionnés pour que les résultats de ces mesures soient optimisés.
On pourra aussi calibrer de la même manière la sonde lorsqu'elle est munie de prolongateurs supplémentaires venant se connecter au niveau des embases 310, 320, 330, en sélectionnant les diamètres et sections des prolongateurs de sorte à diminuer l'amortissement.
D'autre part, les matériaux choisis pour les sondes, tels que le PVC, le silicone, le polyuréthane, un élastomère, un PET, PEBA (Polyéther Bloc Amide) ou tout autre type de matériau, ainsi que les épaisseurs de parois et leur dureté choisies, permettent d'avoir une rigidité satisfaisante tout en ayant une finesse de paroi, ce qui permet de conserver un diamètre extérieur relativement faible.
Enfin, la configuration particulière de la sonde selon l'invention dans sa zone distale 9 (telle que représentée dans les figures 11, 12 et 13) permet d'obtenir une rigidité facilitant l'introduction de la sonde dans l'urètre, dans le passage de la barrière biologique naturelle qu'est le sphincter vésical, et jouant un rôle dans le faible amortissement mesuré par la Demanderesse.

**Revendications**

1. Sonde destinée à un examen urodynamique d'un patient, comprenant une partie proximale (60), et une partie distale (50) s'étendant longitudinalement de sorte à être logée dans l'urètre du patient et sur une longueur suffisante pour qu'une zone d'extrémité soit localisée dans la vessie du patient, la partie distale comprenant trois canaux internes indépendants (10, 20, 30), **caractérisée en ce qu'**elle comprend, dans sa partie distale (50), un tube intérieur (120) et un tube extérieur (110), au moins une partie du tube intérieur (120) s'étendant longitudinalement dans le tube extérieur (110) de sorte à définir entre eux un premier canal (10), les deux autres canaux (20, 30) s'étendant dans le tube intérieur (120).

2. Sonde selon la revendication précédente, **caractérisée en ce que** la surface intérieure du tube extérieur (110) et la surface externe du tube intérieur (120) sont en moyenne suffisamment éloignées l'une de l'autre pour qu'un fluide perfusé puisse circuler dans le premier canal (10) avec une pression apte à servir une mesure urodynamique.

3. Sonde selon l'une des deux revendications précédentes, **caractérisée en ce que** le tube extérieur (110) est agencé de sorte que le premier canal (10) débouche, en opération, au niveau d'une zone de l'urètre de sorte qu'un fluide perfusé dans le premier canal (10) puisse exercer une pression sur les parois de l'urètre apte à servir une mesure urodynamique, et **en ce que** le tube intérieur (120) est agencé de sorte que les deux autres canaux (20, 30) débouchent au niveau de la zone d'extrémité (9) de la partie distale (50).

4. Sonde selon la revendication précédente, **caractérisée en ce que** le premier canal (10) débouche par au moins un oeil traversant (11, 12, 13, 14) une paroi du tube extérieur (110).

5. Sonde selon la revendication 3, **caractérisée en ce que** le premier canal (10) débouche par plusieurs oeils (11, 12, 13, 14) traversant la paroi du tube extérieur (110), et **en ce que** ces oeils sont localisés de sorte à répartir une prise de pression sur la totalité de la circonférence de ladite zone de l'urètre.

6. Sonde selon l'une des trois revendications précédentes, **caractérisée en ce que** le tube intérieur (120) est agencé de sorte que le deuxième canal (20) puisse véhiculer un fluide pour remplir la vessie, que le troisième canal (30) puisse déboucher dans la vessie de sorte qu'un fluide perfusé dans le troisième canal (30) puisse exercer une pression sur un fluide remplissant la vessie apte à servir une mesure urodynamique.

7. Sonde selon la revendication précédente, **caractérisée en ce que** le troisième canal (30) débouche par au moins un oeil traversant (31) une paroi du tube intérieur (120).

8. Sonde selon l'une des revendications précédentes, **caractérisée en ce que** le tube intérieur (120) comporte une paroi interne s'étendant longitudinalement au tube intérieur (120) de sorte à définir lesdits deux autres canaux (20, 30).

9. Sonde selon l'une des deux revendications précédentes, **caractérisée en ce qu'**elle comprend en outre un tube intermédiaire (140) s'étendant au moins en partie entre le tube intérieur (120) et le tube extérieur (110) et dont une extrémité (15) ferme le premier canal (10).

10. Sonde selon la revendication précédente, **caractérisée en ce que** le tube intermédiaire (140) s'étend jusqu'à l'extrémité du tube intérieur (120).

11. Sonde selon la revendication précédente, **caractérisée en ce que** le tube extérieur (110) se prolonge sur une partie du tube intermédiaire (140), et **en ce que** la sonde comprend en outre un tube terminal (150) s'étendant sur l'autre partie du tube intermédiaire (140) en prolongement du tube extérieur (110).

12. Sonde selon la revendication 10, **caractérisée en ce que** le tube extérieur (110) se prolonge sur l'ensemble du tube intermédiaire (140).

13. Sonde selon la revendication 9, **caractérisée en ce que** le tube intermédiaire (140) s'étend sur une partie du tube intérieur (120), **en ce que** le tube extérieur (110) s'étend jusqu'à l'extrémité du tube intermédiaire (140), et **en ce que** la sonde comprend un tube terminal (150) fixé sur la partie libre du tube intérieur (120).

14. Sonde selon l'une des revendications précédentes, **caractérisée en ce que** la partie proximale (60) comprend un

**EP 1 850 752 B1**

tube urétral (210), un premier tube vésical (220) et un deuxième tube vésical (230), étant respectivement en communication avec ledit premier canal (10), le deuxième canal (20) et le troisième canal (30).

15. Sonde selon l'une des revendications précédentes, **caractérisée en ce qu'**elle comprend en outre des moyens de connexion (310, 320, 330) reliés en bout de la partie proximale de la sonde, ces moyens de connexion étant agencés de sorte à connecter la sonde avec des appareils de perfusion, de mesure urodynamique ou d'autres dispositifs.

16. Sonde selon l'une des revendications précédentes, **caractérisée en ce qu'**elle est en PVC, silicone, polyuréthane, élastomère, PET, ou PEBA.

17. Sonde selon l'une des revendications précédentes, **caractérisée en ce que** sa partie distale (50) a un diamètre moyen compris entre charrière 7 et charrière 12.

18. Sonde selon l'une des revendications précédentes, **caractérisé en ce qu'**elle comprend en outre un ensemble prolongateur comportant deux canaux de prolongation pour venir se connecter à l'extrémité de la partie proximale (60), agencés pour prolonger deux desdits trois canaux de la sonde destinés respectivement à des mesures urétrales et vésicales.

19. Sonde selon la revendication précédente, **caractérisé en ce que** l'ensemble prolongateur comprend un troisième canal de prolongation, indépendant des deux canaux de prolongation précités, apte à venir se connecter à l'extrémité de la partie proximale du troisième canal.

**Claims**

1. A probe intended for urodynamic examination of a patient, comprising a proximal part (60), and a distal part (50) extending longitudinally such that it is housed in the urethra of the patient and over a sufficient length so that an end zone is located in the bladder of the patient, the distal part comprising three independent inner channels (10, 20, 30), **characterised in that** it comprises, in its distal part (50), an inner tube (120) and an outer tube (110), at least a part of the inner tube (120) extending longitudinally in the outer tube (110) so as to jointly define a first channel (10), the two other channels (20, 30) extending in the inner tube (120).

2. The probe as claimed in the preceding claim, **characterised in that** the inner surface of the outer tube (110) and the outer surface of the inner tube (120) are, on average, sufficiently apart from each other so that a perfused fluid can circulate in the first channel (10) at a pressure capable of acting as urodynamic measurement.

3. The probe as claimed in any one of the two preceding claims, **characterised in that** the outer tube (110) is arranged such that the first channel (10) discharges, when operating, at the level of a zone of the urethra such that a perfused fluid in the first channel (10) can exert pressure on the walls of the urethra capable of acting as urodynamic measurement, and **in that** the inner tube (120) is arranged such that the two other channels (20, 30) discharge at the level of the end zone (9) of the distal part (50).

4. The probe as claimed in the preceding claim, **characterised in that** the first channel (10) discharges via at least one eye passing through (11, 12, 13, 14) a wall of the outer tube (110).

5. The probe as claimed in Claim 3, **characterised in that** the first channel (10) discharges via several eyes (11, 12, 13, 14) passing through the wall of the outer tube (110), and **in that** these eyes are located so as to distribute a pressurising over the entire circumference of said zone of the urethra.

6. The probe as claimed in any one of the three preceding claims, **characterised in that** the inner tube (120) is arranged so that the second channel (20) can convey fluid to fill the bladder, the third channel (30) can discharge into the bladder such that a perfused fluid in the third channel (30) can exert pressure on a fluid filling the bladder capable of acting as urodynamic measurement.

7. The probe as claimed in the preceding claim, **characterised in that** the third channel (30) discharges via at least one eye passing through (31) a wall of the inner tube (120).

8. The probe as claimed in any one of the preceding claims, **characterised in that** the inner tube (120) comprises an inner wall extending longitudinally to the inner tube (120) so as to define said two other channels (20, 30).

9. The probe as claimed in any one of the two preceding claims, **characterised in that** it also comprises an intermediate tube (140) extending at least in part between the inner tube (120) and the outer tube (110) and one end (15) of which closes the first channel (10).

10. The probe as claimed in the preceding claim, **characterised in that** the intermediate tube (140) extends as far as the end of the inner tube (120).

11. The probe as claimed in the preceding claim, **characterised in that** the outer tube (110) extends over a part of the intermediate tube (140), and **in that** the probe also comprises a terminal tube (150) extending over the other part of the intermediate tube (140) in extension of the outer tube (110).

12. The probe as claimed in Claim 10, **characterised in that** the outer tube (110) extends over all of the intermediate tube (140).

13. The probe as claimed in Claim 9, **characterised in that** the intermediate tube (140) extends over a part of the inner tube (120), **in that** the outer tube (110) extends as far as the end of the intermediate tube (140), and **in that** the probe comprises a terminal tube (150) fixed on the free part of the inner tube (120).

14. The probe as claimed in any one of the preceding claims, **characterised in that** the proximal part (60) comprises a urethral tube (210), a first bladder tube (220) and a second bladder tube (230), respectively communicating with said first channel (10), the second channel (20) and the third channel (30).

15. The probe as claimed in any one of the preceding claims, **characterised in that** it also comprises connecting means (310, 320, 330) connected at the end of the proximal part of the probe, these connecting means being arranged so as to connect the probe with perfusion and urodynamic measuring equipment or other devices.

16. The probe as claimed in any one of the preceding claims, **characterised in that** it is made of PVC, silicon, polyurethane, elastomer, PET, or PEBA.

17. The probe as claimed in any one of the preceding claims, **characterised in that** its distal part (50) has an average diameter between charriere 7 and charriere 12.

18. The probe as claimed in any one of the preceding claims, **characterised in that** it also comprises an extension assembly comprising two extension channels for connecting to the end of the proximal part (60), arranged to extend two of said three channels of the probe respectively intended for urethral and bladder measurements.

19. The probe as claimed in the preceding claim, **characterised in that** the extension assembly comprises a third extension channel, independent of the two abovementioned extension channels, capable of connecting to the end of the proximal part of the third channel.

**Patentansprüche**

1. Für eine urodynamische Untersuchung eines Patienten vorgesehene Sonde, aufweisend einen proximalen Teil (60) und einen distalen Teil (50), der sich längs erstreckt, um in der Harnröhre des Patienten aufgenommen zu werden, und eine ausreichende Länge hat, so dass eine Endzone in der Harnblase des Patienten angeordnet ist, wobei der distale Teil drei unabhängige innere Kanäle (10,20,30) aufweist, **dadurch gekennzeichnet, dass** die Sonde in ihrem distalen Teil (50) ein Innenrohr (120) und ein Außenrohr (110) aufweist, wobei sich mindestens ein Teil des Innenrohrs (120) längs in dem Außenrohr (110) erstreckt, um dazwischen einen ersten Kanal (10) zu definieren, wobei sich die zwei anderen Kanäle (20, 30) in dem Innenrohr (120) erstrecken.

2. Sonde nach dem vorstehenden Anspruch, **dadurch gekennzeichnet, dass** die innere Oberfläche des Außenrohrs (110) und die äußere Oberfläche des Innenrohrs (120) im Mittel ausreichend voneinander beabstandet sind, so dass in dem ersten Kanal (10) ein Infusionsfluid bei einem Druck, der geeignet ist, einer urodynamischen Messung zu dienen, zirkulieren kann.

3. Sonde nach einem der zwei vorstehenden Ansprüche, **dadurch gekennzeichnet, dass** das Außenrohr (110) so konstruiert ist, dass in Verwendung der erste Kanal (10) in einer Zone der Harnröhre ausmündet, so dass ein Infusionsfluid in dem ersten Kanal (10) einen Druck auf die Wände der Harnröhre ausüben kann, der geeignet ist, einer urodynamischen Messung zu dienen, und **dadurch**, dass das Innenrohr (120) so konstruiert ist, dass die zwei anderen Kanäle (20,30) in der Endzone (9) des distalen Teils (50) ausmünden.

4. Sonde nach dem vorstehenden Anspruch, **dadurch gekennzeichnet, dass** der erste Kanal (10) über mindestens ein durch eine Wand des Außenrohrs (110) hindurchgehendes Loch (11,12,13,14) ausmündet.

5. Sonde nach Anspruch 3, **dadurch gekennzeichnet, dass** der erste Kanal (10) über mehrere durch die Wand des Außenrohrs (i10) hindurchgehende Löcher (11,12,13,14) ausmündet, und **dadurch**, dass diese Löcher so angeordnet sind, um einen Druckabgriff über den gesamten Umfang der Zone der Harnröhre zu verteilen.

6. Sonde nach einem der drei vorstehenden Ansprüche, **dadurch gekennzeichnet, dass** das Innenrohr (120) so konstruiert ist, dass der zweite Kanal (20) ein Fluid transportieren kann, um die Harnblase zu füllen, **dadurch**, dass der dritte Kanal (30) in der Harnblase ausmünden kann, so dass ein Infusionsfluid in dem dritten Kanal (30) einen Druck auf ein die Harnblase füllendes Fluid ausüben kann, das geeignet ist, einer urodynamischen Messung zu dienen.

7. Sonde nach dem vorstehenden Anspruch, **dadurch gekennzeichnet, dass** der dritte Kanal (30) über mindestens ein durch eine Wand des Innenrohrs (120) hindurchgehendes Loch (31) ausmündet.

8. Sonde nach einem der vorstehenden Ansprüche, **dadurch gekennzeichnet, dass** das Innenrohr (120) eine Innenwand aufweist, die sich längs zu dem Innenrohr (120) erstreckt, um so die zwei anderen Kanäle (20,30) zu definieren.

9. Sonde nach einem der zwei vorstehenden Ansprüche, **dadurch gekennzeichnet, dass** sie außerdem ein Zwischenrohr (140) aufweist, das sich zumindest teilweise zwischen dem Innenrohr (120) und dem Außenrohr (110) erstreckt und ein Ende dessen (15) den ersten Kanal (10) schließt.

10. Sonde nach dem vorstehenden Anspruch, **dadurch gekennzeichnet, dass** sich das Zwischenrohr (140) bis zu dem Ende des Innenrohrs (120) erstreckt.

11. Sonde nach dem vorstehenden Anspruch, **dadurch gekennzeichnet, dass** sich das Außenrohr (110) über einen Teil des Zwischenrohrs (140) erstreckt, und **dadurch**, dass die Sonde außerdem ein Rohrendstück (150) aufweist, das sich in Verlängerung des Außenrohrs (110) über den anderen Teil des Zwischenrohrs (140) erstreckt.

12. Sonde nach Anspruch 10, **dadurch gekennzeichnet, dass** sich das Außenrohr (110) über das gesamte Zwischenrohr (140) erstreckt.

13. Sonde nach Anspruch 9, **dadurch gekennzeichnet, dass** sich das Zwischenrohr (140) über einen Teil des Innenrohrs (120) erstreckt, **dadurch**, dass sich das Außenrohr (110) bis zu dem Ende des Zwischenrohrs (140) erstreckt, und **dadurch**, dass die Sonde ein an dem freien Teil des Innenrohrs (120) angebrachtes Rohrendstück (150) aufweist.

14. Sonde nach einem der vorstehenden Ansprüche, **dadurch gekennzeichnet, dass** der proximale Teil (60) ein Harnröhre-Rohr (210), ein erstes Harnblase-Rohr (220) und ein zweites Harnblase-Rohr (230) aufweist, die jeweils in Verbindung mit dem ersten Kanal (10), dem zweiten Kanal (20) und dem dritten Kanal (30) sind.

15. Sonde nach einem der vorstehenden Ansprüche, **dadurch gekennzeichnet, dass** sie außerdem Anschlussmittel (310,320,330) aufweist, die mit dem Ende des proximalen Teils der Sonde verbunden sind, wobei diese Anschlussmittel vorgesehen sind, um die Sonde an Infusionsgeräte, Geräte für urodynamische Messungen oder andere Vorrichtungen anzuschließen.

16. Sonde nach einem der vorstehenden Ansprüche, **dadurch gekennzeichnet, dass** sie aus PVC, Silikon, Polyurethan, Elastomer, PET oder PEBA ist.

17. Sonde nach einem der vorstehenden Ansprüche, **dadurch gekennzeichnet, dass** ihr distaler Teil (50) einen durchschnittlichen Durchmesser zwischen 7 Charrière und 12 Charrière hat.

18. Sonde nach einem der vorstehenden Ansprüche, **dadurch gekennzeichnet, dass** sie außerdem eine Verlängerungseinrichtung aufweist, die zwei mit dem Ende des proximalen Teils (60) zu verbindende Verlängerungskanäle aufweist, die dazu dienen, zwei der drei Kanäle der Sonde zu verlängern, die jeweils für Harnröhre- und Harnblase-Messungen vorgesehen sind.

19. Sonde nach dem vorstehenden Anspruch, **dadurch gekennzeichnet, dass** die Verlängerungseinrichtung einen dritten Verlängerungskanal aufweist, der von den vorstehend erwähnten zwei Kanälen unabhängig ist und mit dem Ende des proximalen Teils des dritten Kanals verbunden werden kann.

EP 1 850 752 B1

FIG.1

FIG.2

FIG.3

15

FIG.4

FIG.5

FIG.6

FIG.7

FIG.8a

FIG.8b

FIG.9a

FIG.9b

FIG.10a

FIG.10b

FIG.10c

FIG.10d

**FIG.10e**

**FIG.10f**

**FIG.11**

**FIG.12**

**FIG.13**

FIG.14

FIG.15

FIG.16

FIG.17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5385563 A
- EP 0878166 A
- GB 2123300 A

**Littérature non-brevet citée dans la description**

- **Braun ; Wickham.** The uretral pressure profile. *British Journal of Urology,* 1969, vol. 41, 211.216